# FASCICULE DE BREVET EUROPEEN

(11) **EP 1 850 887 B1**
(45) Date de publication et mention de la délivrance du brevet: **09.07.2008**
(21) Numéro de dépôt: 06709261.9
(22) Date de dépôt: 07.02.2006
(51) Int. Cl.: A61L 9/04, A61K 8/11, A61L 9/014

(54) **COMPOSITION PARFUMEE A DIFFUSION CONTROLEE**
PARFÜMIERTE ZUSAMMENSETZUNG MIT KONTROLLIERTER FREISETZUNG
CONTROLLED-RELEASE PERFUMED COMPOSITION

(30) Priorité: 14.02.2005 FR 0501442
(43) Date de publication de la demande: 07.11.2007
(73) Titulaire: Cervilla, Monique, 31400 Toulouse (FR)
(72) Inventeur: Cervilla, Monique, 31400 Toulouse (FR)
(74) Mandataire: Ravina, Bernard
(86) Numéro de dépôt international: PCT/FR2006/000273
(87) Numéro de publication internationale: WO 2006/084985

(56) Documents cités:
- EP-A- 0 317 658
- US-A- 3 661 838
- US-A- 5 500 223
- US-A1- 2003 013 632
- US-A1- 2005 003 996

## Description

La présente invention se rapporte au domaine de la diffusion de substances odorantes, et plus particulièrement à des compositions associant un parfum et son support, utilisées dans le domaine cosmétique ou la parfumerie.

La présente invention a pour objet une composition comprenant essentiellement une substance odorante élaborée, telle qu'un parfum, apte à assurer une diffusion progressive et différenciée des composants de la substance odorante. La présente invention a également pour objet un procédé de préparation d'une telle composition, ainsi qu'un dispositif pour la sa préparation extemporanée.

Un parfum en tant que message olfactif ne constitue pas une odeur unique, mais est composé d'une multitude d'odeurs qui sont perçues simultanément et/ou successivement, et que les parfumeurs ont l'habitude de classer en trois groupes, appelés note de fond, note de coeur et note de tête.

Les senteurs lourdes durables constituent la note de fond. Elle comprend les fragrances musquées, ambrées, épicées, ou balsamiques. La volatilité de cette note est relativement faible, aussi sa persistance olfactive est largement supérieure à 4 heures et peut durer jusqu'à 24 heures, voire plusieurs jours. La palette des notes de coeur est représentée par toutes les senteurs chaudes, fleuries, sucrées, dont la durée de volatilisation s'échelonne entre 2 et 4 heures. La note de tête représente la classe la plus variée, comprenant les senteurs légères, fraîches, vertes et fruitées, offrant aux parfumeurs les possibilités de variations olfactives les plus larges, mais dont la volatilité est dramatiquement élevée : elles sont totalement évaporées en 15 à 30 minutes.

Le défi pour les parfumeurs est donc de prolonger la durée de vie de la note de tête, tout en conservant l'équilibre de la composition parfumée. Depuis de nombreuses années, des tentatives ont été faites pour ralentir l'évaporation des substances odorantes. Parmi les plus notables, on citera l'adsorption dans des zéolites ou du carbone, ou la complexation dans des cyclodextrines pour prolonger la fragrance dans des produits cosmétiques ou dans des désodorisants. Dans ce dernier cas, des particules de très petite taille sont introduites dans une solution parfumée et sont pulvérisées ou appliquées avec le cosmétique, de sorte qu'elles adhèrent à la peau. Une autre solution a été de préparer des solutions parfumées en forçant la concentration de la note de tête de façon à ce qu'elle soit toujours perceptible à partir des particules où elles sont piégées.

Ces techniques présentent cependant de sévères limitations car elles ciblent une famille de composés et sont inadaptées lorsque la substance odorante est un mélange complexe de composés dont les vitesses de diffusion sont très différentes. Or une réduction de la vitesse de diffusion des notes de coeur et de tête peut conduire à une absence totale de diffusion de la note de fond. La fragrance perçue ne correspond plus en rien à la création voulue par l'homme de l'art, selon un certain équilibre.

La présente invention se propose de remédier à ces inconvénients grâce à l'emploi d'un support de parfum composé de deux ou de plusieurs lots de particules ayant des propriétés de rétention différentes, de sorte que les composés les plus volatils comme les composés les plus lourds soient libérés en permanence par l'un ou par l'autre de ces lots de particules durant une période de temps étendue. La mise au point d'un tel support, appelé support complexe, a nécessité de surmonter un certain nombre d'obstacles liés notamment aux caractéristiques physico-chimiques des particules du support et des composés du parfum qui devaient être associés.

Dans un premier temps il a été imaginé d'utiliser des lots de particules microporeuses dont les propriétés de rétention se distinguent du fait d'un diamètre des particules et d'une taille de pores variables. Quand on examine les possibilités de modulation de ces paramètres, on constate que la taille des pores a une contribution relativement modeste comparée au diamètre des particules. Il semblait donc possible de régler la vitesse de diffusion par la taille des particules. En réalité, cette idée conduit à une impasse technologique. En effet, les différences de points d'ébullition entre les composés de la note de tête et ceux de la note de fond sont telles qu'il serait nécessaire d'utiliser des particules de diamètres très élevés et très faibles. Or, la manipulation de particules sèches de très petits diamètres pose des problèmes pratiques insurmontables notamment de pulvérulence et d'agrégation. La variété des tailles dans le mélange conduit en outre à une séparation en couches de granulométrie voisine.

De manière surprenante, il a été trouvé qu'il était possible, en utilisant un mélange de supports poreux et pelliculaires, dont les particules ont une taille choisie dans une gamme assez étroite pour éviter les problèmes précédemment exposés, d'obtenir un échelonnement de la rétention des composés d'un parfum permettant la diffusion simultanée et prolongée des notes de tête, de coeur et de fond. Chaque note olfactive est ainsi perçue durablement.

Les supports pelliculaires sont des supports anisotropes qui présentent une partie centrale non poreuse entourée par une étroite couronne poreuse. On a constaté que les supports pelliculaires se comportent, du point de vue de la diffusion, comme des supports poreux de très petit diamètre. Par exemple, un support pelliculaire avec une couronne externe de 1 micron se comporte comme un support complètement poreux de 2 microns de diamètre. La surface spécifique des supports pelliculaires est bien entendu faible par rapport à celle des supports complètement poreux mais elle est beaucoup plus importante que celle des supports non poreux et nous avons montré qu'elle était parfaitement adaptée à la diffusion optimale des fragrances lourdes.

Ainsi, la présente invention a pour objet une composition parfumée à diffusion contrôlée comprenant:
a) un parfum, et
b) un support complexe susceptible de se charger en ledit parfum, comprenant en mélange au moins :
   - i) un premier lot de particules, dites particules poreuses, ayant un diamètre moyen compris entre 10 µm et 500 µm, dont au moins 80% du volume est poreux, et
   - ii) un deuxième lot de particules, dites particules pelliculaires, ayant un diamètre moyen compris entre 10 µm et 500 µm, dont seule une couche externe représentant au plus 20% du volume est poreuse.

Le terme parfum s'applique à toute matière odoriférante sous forme libre, telles que les huiles essentielles, les extraits végétaux, les composés chimiques odoriférants, pris seuls ou en mélange, ainsi que les dilutions alcooliques ou hydro-alcooliques de ces préparations. Plus généralement, sont concernées toutes les matières premières connues, naturelles et synthétiques, qu'une personne qualifiée dans l'art de la parfumerie est susceptible d'utiliser pour composer un parfum. Il est important de remarquer que la composition selon l'invention n'entend pas sélectionner un composant odorant plutôt qu'un autre, ni se limiter à certains types de compositions parfumées, puisque c'est le choix du support particulaire qui est déterminant pour assurer une diffusion progressive, quel que soit le parfum associé.

Ainsi dans la composition selon l'invention, le parfum peut être notamment choisi parmi un extrait naturel brut, une huile essentielle, une composition parfumée naturelle, une composition parfumée synthétique ou un mélange de ceux-ci.

Le support de parfum de la composition selon l'invention est composé d'au moins deux lots de particules. Un troisième lot, ayant des caractéristiques physico-chimiques intermédiaires, peut être ajouté au support ici décrit, sans que le principe général de l'invention soit modifié.

Cependant pour des raisons de clarté et de simplicité, la présente description se limitera à un support comprenant deux lots de particules. Ceci est d'ailleurs amplement justifié du fait que les résultats obtenus avec les supports binaires décrits sont tout à fait satisfaisants.

Comme déjà indiqué, le support de parfum comprend en mélange au moins deux lots de particules, dont la taille, repérée par le diamètre, est dans un ordre de grandeur de 10 µm à 500 µm, mais dont la porosité est différente. Les particules dites poreuses, présentent une porosité dans au moins 80% de leur volume et le plus communément dans la totalité de leur volume, tandis que les particules dites pelliculaires, ont seulement une couche externe poreuse, celle-ci représentant au plus 20% du volume.

Selon une caractéristique préférée, les particules poreuses dudit premier lot ont un diamètre compris entre 50 et 200 µm. Selon une autre caractéristique préférée, les particules pelliculaires dudit deuxième lot ont un diamètre compris entre 15 µm et 150 µm.

Les caractéristiques de diamètre et de porosité ci-dessus permettent de répondre en grande partie aux conditions recherchées. Pour s'approcher mieux encore des conditions souhaitées et assurer une diffusion équilibrée du parfum, il est avantageux de sélectionner des particules dont la surface spécifique, correspondant à une capacité de charge, se situe dans un intervalle donné.

De manière avantageuse, on choisit les particules poreuses dudit premier lot telles qu'elles ont une surface spécifique comprise entre 100 m²/g et 800 m²/g, de préférence entre 300 m²/g et 800 m²/g, et encore de préférence entre 500 m²/g et 800 m²/g. De manière également avantageuse, les particules pelliculaires dudit deuxième lot ont une surface spécifique comprise entre 1 m²/g et 100 m²/g, de préférence entre 10 m²/g et 100 m²/g.

Les particules du support peuvent être réalisées à partir de différents matériaux, tels que des polymères synthétiques ou naturels, ou à partir de matériaux inorganiques. Ces matériaux doivent être inertes vis-à-vis du parfum mais aptes à se lier à lui par des interactions réversibles. Ils peuvent présenter par eux-mêmes les propriétés requises en terme de porosité et d'affinité chimique, ou peuvent subir des transformations physico-chimiques leur conférant lesdites propriétés. D'une manière particulièrement préférée, dans la composition selon l'invention, les particules composant ledit support sont choisies parmi les billes de silice poreuse, les billes de verre modifiées, les fibres de verre, les particules de cellulose ou les fibres de cellulose.

Le terme "particule" désigne des fragments de matière de petite taille, dont la forme peut être sensiblement régulière, comme dans le cas de billes ou de fibres, ou quelconque lorsque la matière a été par exemple broyée. Bien que la présente description utilise une terminologie habituellement réservée aux particules sphériques, en particulier la notion de diamètre, il est entendu qu'elle s'étend à toutes les formes de particules, pour lesquelles l'homme du métier sait calculer des grandeurs équivalentes.

De manière inattendue, il a été trouvé que les billes de silice poreuses, telles que celles utilisées pour la fabrication des colonnes de chromatographie pouvaient être utilisées pour la préparation des supports poreux selon l'invention. Un large choix de telles particules de silice de diverses tailles est disponible dans le commerce.

Il a été trouvé par ailleurs que des billes de verre traitées de manière à créer une couronne poreuse de faible épaisseur à leur surface, telles que celles utilisées en tant que pré-filtre des colonnes de chromatographie, peuvent être avantageusement utilisées comme particules pelliculaires pour réaliser la composition selon la présente invention. Ces particules pelliculaires en silice, neutres ou greffées par des substituants hydrophobes, sont également disponibles dans le commerce. Cependant, celles-ci ne présentent pas toute la gamme de diamètres et de surfaces spécifiques souhaités. Il est donc intéressant de pouvoir les préparer à partir de billes de verre lisses calibrées. Une méthode reposant sur une attaque à l'acide fluorhydrique est détaillée plus loin en exemple.

Pour favoriser l'imprégnation du parfum dans la porosité, les particules composant ledit support complexe sont de préférence hydrophobes. Les particules poreuses ou pelliculaires peuvent être rendues hydrophobes par dérivation sur les groupements hydroxyles présents en surface de la silice. Les substituants préférés sont les groupements alkyles linéaires, saturés et ramifiés, notamment C1-C18.

Des particules d'acétate de cellulose, ou des particules de cellulose microcristalline qui peuvent être rendues hydrophobes par différentes méthodes, par exemple par acylation, peuvent aussi être employées comme support poreux.

Les deux lots de particules, poreuses et pelliculaires, peuvent entrer dans la composition selon l'invention en quantités égales. Dans la mesure où les particules pelliculaires ont une capacité de charge moindre que les particules poreuses, il est néanmoins judicieux d'apporter les deux lots dans des proportions inégales. Selon une caractéristique intéressante de la présente invention, ledit support complexe comprend ledit premier lot de particules poreuses et ledit deuxième lot de particules pelliculaires dans un rapport en poids compris entre 1/1 et 1/50, de préférence entre 1/5 et 1/30, de manière encore préférée entre 1/10 et 1/20.

La composition sèche parfumée selon l'invention est conçue de sorte que l'intégralité du parfum est absorbé par le support. C'est pourquoi, selon une caractéristique particulière, la composition comprend de 10 à 90%, de support, en poids rapporté au poids total de la composition. De préférence la composition comprend de 20 à 80%, et de manière encore préférée de 40 à 75% de support.

De manière quasi symétrique, la composition selon l'invention comprend de 10 à 90% de parfum, en poids rapporté au poids total de la composition. De préférence elle comprend de 20 à 80 %, et de manière encore préférée de 25 à 60% de parfum. Ces proportions sont définies à l'instant initial de préparation de la composition, avant que le solvant du parfum ne s'évapore.

Bien entendu, la concentration initiale du parfum joue un rôle, puisqu'on peut utiliser pour préparer la composition, un extrait concentré aussi bien qu'une eau de toilette. Quoi qu'il en soit, la plupart des solvants employés en parfumerie sont très volatils et s'évaporent rapidement Ainsi, selon la concentration initiale du parfum, la composition selon l'invention contiendra, dix minutes environ après la réalisation de la composition, moins de 25%, de préférence moins de 15%, et encore de préférence moins de 5% d'un solvant volatil, en poids de solvant rapporté au poids initial de parfum.

Est également objet de la présente invention un procédé de préparation d'une composition parfumée sèche, très simple, ne nécessitant ni équipement spécialisé, ni montée en température, ni élimination de solvants. Ledit procédé comprend essentiellement les étapes consistant à:
- préparer un support complexe en mélangeant:
   - i) un premier lot de particules, dites particules poreuses, ayant un diamètre moyen compris entre 10 µm et 500 µm, dont au moins 80% du volume est poreux, et
   - ii) un deuxième lot de particules, dites particules pelliculaires, ayant un diamètre moyen compris entre 10 µm et 500 µm, dont seule une couche externe représentant au plus 20% du volume est poreuse,
- verser un parfum sur ledit support,
- laisser le parfum imprégner le support quelques minutes à température ambiante, et
- placer la composition parfumée sèche ainsi obtenue dans un réceptacle adapté au stockage ou à une utilisation immédiate.

De manière avantageuse, dans le procédé selon l'invention, la préparation dudit support complexe est réalisée à l'aide de deux lots de particules aptes à la préparation d'une composition parfumée sèche, telle que décrite précédemment, et dont les caractéristiques sont ici incluses.

Dans un mode préféré de mise en oeuvre du procédé, ledit premier lot de particules poreuses et ledit deuxième lot de particules pelliculaires sont apportés au mélange dans un rapport en poids compris entre 1/1 et 1/50, de préférence entre 1/5 et 1/30, de manière encore préférée entre 1/10 et 1/20. Les deux lots sont brassés jusqu'à obtenir une répartition homogène des particules à l'aide de moyens mécaniques communément utilisés à cette fin et connus de l'homme de l'art.

La deuxième étape du procédé selon l'invention consiste à verser le parfum sur le support particulaire, puis dans une troisième étape à laisser le parfum imprégner le support. De préférence on utilise pour cela des quantités relatives de support et de parfum telles que :
- ledit support constitue de 10 à 90%, de préférence de 20 à 80%, de manière encore préférée de 40 à 75%, en poids rapporté au poids total de la composition,
- ledit parfum constitue de 10 à 90%, de préférence de 20 à 80 %, de manière encore préférée de 25 à 60% de parfum, en poids rapporté au poids total de la composition, le parfum pouvant contenir dix minutes après la préparation de la composition, moins de 25%, de préférence moins de 15%, et encore de préférence moins de 5% d'un solvant volatile, en poids de solvant rapporté au poids initial de parfum.

De manière particulièrement avantageuse et souhaitée, selon le procédé revendiqué, la proportion de parfum et de support est telle que, après l'étape d'imprégnation, la composition se présente sous la forme d'une poudre sèche odorante.

La composition selon l'invention peut être proposée sous forme d'un dispositif destiné à la préparation extemporanée d'une composition parfumée sèche à diffusion contrôlée comprenant :
- une pluralité de premiers récipients contenant un parfum, et
- un pluralité de deuxièmes récipients contenant un support complexe susceptible de se charger en ledit parfum, comprenant en mélange au moins :
   - i) un premier lot de particules, dites particules poreuses, ayant un diamètre moyen compris entre 10 µm et 500 µm, dont au moins 80% du volume est poreux, et
   - ii) un deuxième lot de particules, dites particules pelliculaires, ayant un diamètre moyen compris entre 10 µm et 500 µm, dont seule une couche externe représentant au plus 20% du volume est poreuse.

   Bien entendu les premiers et deuxièmes récipients du dispositif selon l'invention sont tout particulièrement destinés à contenir respectivement les composants a) c'est-à-dire un parfum, et b) c'est-à-dire un support particulaire complexe, d'une composition parfumée sèche telle que décrite précédemment. Ils sont choisis de sorte qu'ils soient commodes à remplir, à stocker et à ouvrir.
   De manière intéressante, lesdits premiers et deuxièmes récipients contiennent respectivement les quantités de parfum et de support nécessaires à la préparation extemporanée d'une dose de ladite composition sèche parfumée. Ainsi, l'utilisateur peut parfaitement préparer la composition parfumée sèche au moment et à l'endroit où il le souhaite.
   La composition selon la présente invention est destinée à une grande variété d'applications mais est plus particulièrement conçue pour être introduite dans toutes sortes de boîtiers ou d'accessoires en vue de parfumer la peau, les cheveux, les vêtements, les accessoires vestimentaires, les bijoux, ou l'atmosphère d'une pièce. Elle peut être introduite dans un boîtier non hermétique à l'air ou dans un sachet en film polymère finement perforé.
   Les exemples suivants ont vocation à illustrer des modes de réalisation particuliers de l'invention, sans en réduire la portée et sans limiter ses innombrables utilisations possibles.

### EXEMPLE 1 - Composition parfumée sèche sur billes de verre

La composition C1 a été préparée à partir des éléments suivants :
- Le lot de particules poreuses L1 est constitué de billes de silice greffées par 20 % en masse de groupements C18, commercialisées par Fluka sous la référence 60754 (Groupe SIGMA-ALDRICH- FLUKA). Le diamètre des particules est compris dans l'intervalle 63-200 µm, les pores ont un diamètre de 100Å, la porosité occupe 100% du volume des particules. La surface spécifique des particules est de 600 m²/g.
- Le lot de particules pelliculaires L2 est constitué de billes de verre traitées en surface selon le protocole décrit à l'exemple 4 ci-après. Les billes sont greffées par 2% en masse de groupements C18. Leur diamètre moyen est de 25 µm, la porosité forme une couronne d'une épaisseur de 0,5µm. La surface spécifique des particules est de 35 m²/g.
- Le parfum utilisé est L'instant de Guerlain^{™}, qui est une eau de parfum concentrée à 20% dans l'éthanol.
Les lots L1 et L2 ont été préalablement testés pour leur capacité de charge vis-à-vis du parfum choisi. Le lot L1 a une capacité de charge de 100%, (100 g de particules poreuses peuvent absorber 100 g de parfum), le lot L2 a quant à lui une capacité de charge de 10%.

Lorsqu'on apporte 1 g de parfum sur 1 g de support poreux, 0,8 g de solvant s'évapore dans les premières dix minutes, et on obtient 1,2 g de particules poreuses chargées de parfum concentré. Lorsqu'on apporte 0,1 g de parfum sur 1 g de support pelliculaire, 0,08g de solvant s'évapore, et on obtient 1,02 g de particules pelliculaires chargées de parfum concentré.

### EXEMPLE 2 - Procédé de préparation de la composition C1

La composition C1 décrite à l'exemple 1 a été préparée selon le mode opératoire suivant.

On mélange les lots de supports L1 et L2, à raison de 0,2 g de particules L1 et 2 g de particules L2. Puis on ajoute 1 g d'eau de parfum et on laisse imprégner deux minutes à température ambiante. Le solvant alcoolique s'évapore presque totalement dans ce laps de temps et la composition est prête à l'emploi. Si elle n'est pas utilisée immédiatement, elle est conditionnée en sachets ou flacons étanches. Pour un emploi immédiat, elle est placée dans un accessoire diffuseur.

### EXEMPLE 3 - Composition parfumée sèche sur billes de verre et fibres de cellulose

La composition C2 a été préparée à partir des éléments suivants :
- Le lot de particules poreuses L1 est constitué de fibres de docodécylcellulose préparées par acylation de cellulose microcristalline comme décrit à l'exemple 5. Le diamètre moyen des particules est de 100 µm, la porosité occupe 100% du volume des particules.
- Le lot de particules pelliculaires L2 est constitué de billes de verre traitées en surface selon le protocole décrit à l'exemple 4 ci-après. Les billes sont greffées par 2% en masse de groupements C18. Leur diamètre moyen est de 25 µm, la porosité forme une couronne d'une épaisseur de 0,5µm. La surface spécifique des particules est de 35 m²/g.
- Le parfum utilisé est J'adore DIOR^{™}, qui est une eau de parfum concentrée à 20% dans l'éthanol.

Les supports sont mélangés à raison de 0,2 g de support poreux et 2 g de support pelliculaire, puis imprégnés de 1 g de parfum deux minutes à température ambiante. La composition est prête à l'emploi.

### EXEMPLE 4 - Synthèse de supports pelliculaires hydrophobes en verre

La synthèse de deux supports pelliculaires a été réalisée à partir de billes de verre lisses de 25 µm et de 50 µm calibrées par tamisage.

Dans une première étape, on crée une couronne poreuse. Pour cela 10 g de billes et 50 ml d'une solution aqueuse d'acide fluorhydrique à 1% sont introduits dans un flacon de polypropylène bouché. Après incubation deux heures sous agitation, la préparation est neutralisée à la soude et rincée à l'eau. Les particules obtenues ont un diamètre moyen identique à celui des billes lisses de départ, avec toutefois une légère augmentation de la dispersion.

Dans une seconde étape, on procède au greffage par des dérivés acylés. Les particules séchées (10 g) sont introduites dans 50 ml de toluène. On ajoute 0,1 ml de pyridine et 1 g de réactif d'acylation (en excès). Cette préparation est placée une nuit à température ambiante, sous agitation. Elle est ensuite décantée, rincée dans du toluène puis dans du méthanol, et séchée.

Le réactif d'acylation peut être le chloro-diméthyloctadécylsilane (Fluka, réf. 40950) pour une dérivation en C18, le chloro-décyldiméthylsilane (Fluka, réf. 30695) pour une dérivation en C10, ou le chloro-triméthylsilane (Fluka, réf. 92361) pour une dérivation en C1.

Les supports pelliculaires obtenus ont un diamètre moyen de 25µm et 50 µm pour une surface spécifique de 35 m²/g et 17 m²/g, respectivement.

### EXEMPLE 5 - Synthèse de supports poreux hydrophobes cellulosiques

La synthèse de deux supports poreux hydrophobes est réalisée à partir de fibre de cellulose microcristalline du commerce, le premier support ayant une taille de particules d'environ 100µm (AVICEL, réf. PH 200) et le second support ayant une taille de particules d'environ 50µm (AVICEL, PH 101). Chaque échantillon est traité de la même manière pour greffer le groupement acyle C22.

La cellulose est préalablement déshydratée dans du méthanol, puis filtrée et séchée sous vide. Elle est ensuite traitée avec 20% (m/m) de chlorure de stéaryle (FLUKA, Réf. 85727) en présence d'éther de pétrole. Celui-ci est ensuite évaporé et la cellulose est placée dans une étuve à 100°C ventilée (700 mbar) pendant 30 minutes. Enfin, la cellulose est lavée plusieurs fois à l'acétone puis à l'éthanol.

### EXEMPLE 6 - Détermination de l'évaporation des composés de chaque note

### 1) - Principe

La méthode de détermination de l'évaporation d'un parfum repose sur la mesure de la quantité de parfum restant dans un échantillon mis en contact avec l'atmosphère, en fonction du temps. En chromatographie en phase gazeuse (CPG), la position d'un pic sur le chromatogramme est caractéristique d'un composé donné, et l'évolution de sa surface au cours du temps correspond à une variation de concentration dudit composé.

### 2) - Étalonnage du chromatographe

Une première étape est réalisée sur un parfum seul (sans support), selon un protocole opératoire qui sera utilisé pour toutes les autres analyses. Elle permet d'établir la correspondance entre les temps de rétention des composés sur la colonne CPG et les temps d'évaporation définissant les trois notes du parfumeur.

Pour ce faire, on analyse les échantillons de parfum en cours d'évaporation à différents intervalles de temps et classe les pics d'après leur surface : les pics ayant disparu en moins d'une demi-heure sont considérés correspondre à des composés de la note de tête, les pics ayant disparu entre 30 minutes et 4 heures sont considérés correspondre à des composés de la note de coeur, et les pics ayant disparu en plus de 4 heures sont considérés correspondre à des composés de la note de fond.

On repère alors la position de ces 3 groupes de pics sur les chromatogrammes et on détermine les 3 intervalles de temps de rétention sur la colonne CPG dans les conditions de l'analyse choisies, correspondant à chaque note. On n'identifie pas les composés, mais on les classe en fonction de leur vitesse d'évaporation.

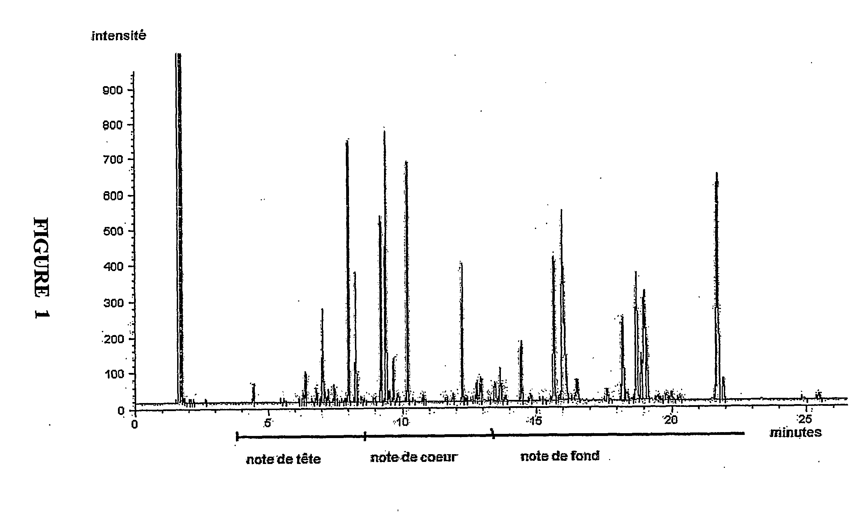

La Figure 1 est un chromatogramme obtenu à t_{O}, sur lequel tous les composés apparaissent. Dans les conditions choisies, les composés de la note de tête sont détectés entre 4 et 9 mn, les composés de la note de coeur sont détectés entre 9 et 13 mn, et les composés de la note de fond sont détectés à partir de 13 mn et jusqu'à 25 mn.

### 3) - Établissement de la cinétique d'évaporation d'un parfum

Des échantillons de parfum sont analysés après des durées d'évaporation plus ou moins longues. L'analyseur calcule l'évolution des surfaces de pics pour les trois intervalles de rétention définis ci-dessus. La cinétique d'évaporation d'une note est la courbe représentant la proportion non évaporée de la note en question (% résiduel) en fonction de la durée d'évaporation de l'échantillon.

### EXEMPLE 7 - Cinétique d'évaporation sur supports de verre modifié + silice

Cet exemple vise à illustrer l'effet d'un support binaire verre + silice sur le rythme de diffusion des différentes notes d'un parfum, ici L'instant de Guerlain^{™}.

Quatre échantillons ont été étudiés.
- le premier échantillon est la composition C1 préparée selon l'Exemple 1 à partir du support binaire L1 + L2, imprégné du parfum;
- le deuxième échantillon est le parfum déposé sur un support ne comprenant que le lot de particules poreuses L1;
- le troisième échantillon est le parfum déposé sur un support ne comprenant que le lot de particules pelliculaires L2;
- l'échantillon témoin est le parfum seul, (sans support), placé dans les mêmes conditions de température que les trois premiers échantillons.

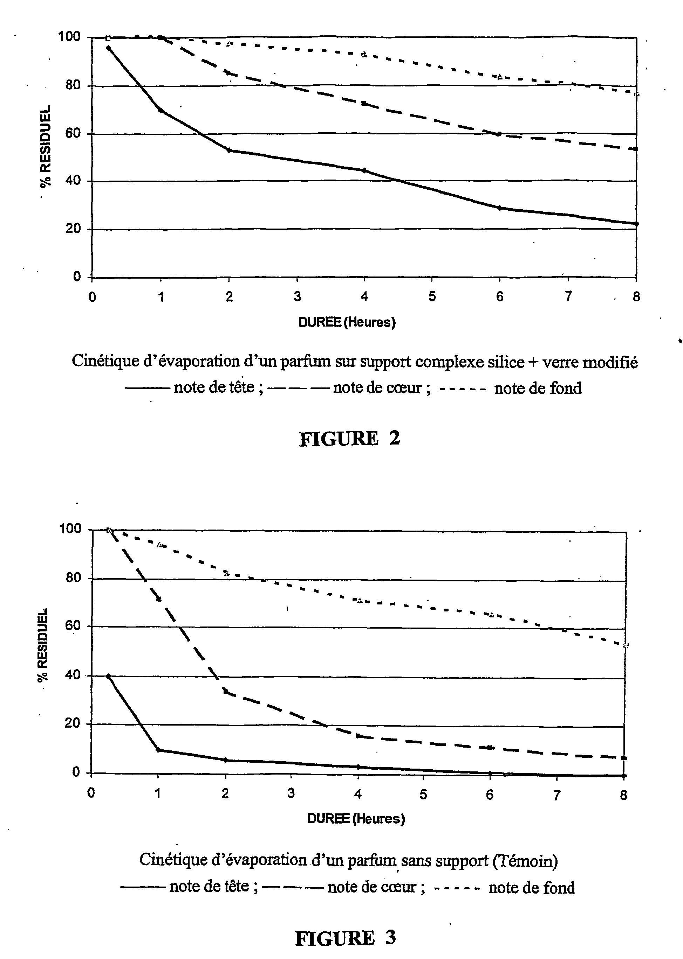

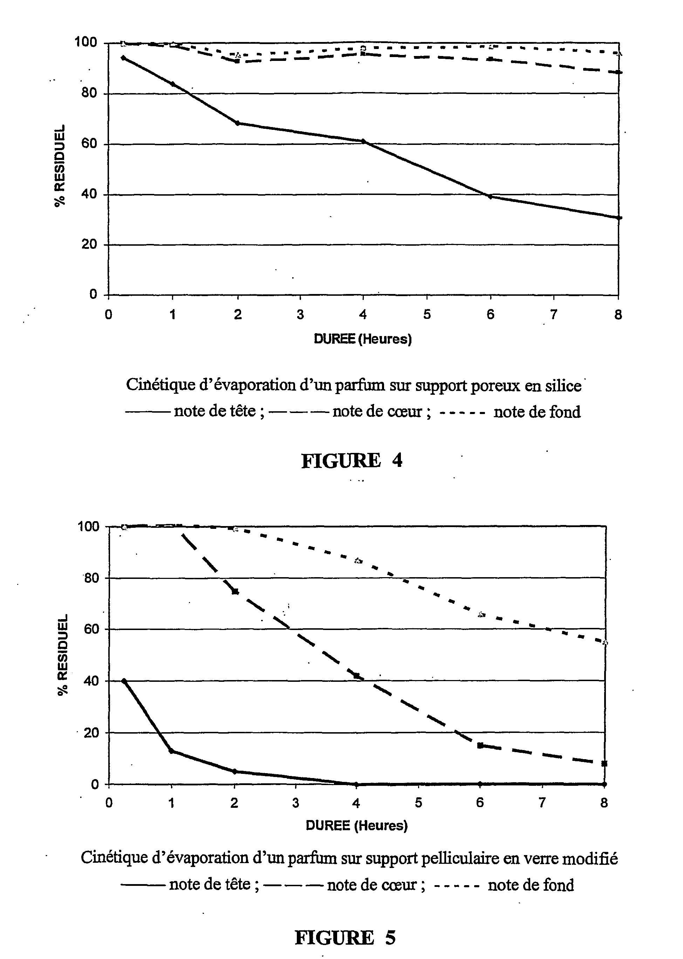

Les résultats obtenus sont présentés sur les figures 2, 3 4 et 5. Pour la composition binaire C1, les trois notes sont encore présentes après huit heures (Figure 2) alors que pour la composition témoin, les deux notes les plus légères ont quasiment disparu après le même laps de temps (Figure 3). La contribution des supports poreux et pelliculaire au contrôle de la diffusion du parfum est illustrée par les Figures 4 et 5, respectivement

### EXEMPLE 8 - Cinétique d'évaporation sur supports de verre + cellulose

Cet exemple vise à illustrer l'effet d'un second support binaire sur le rythme de diffusion des différentes notes du parfum J'adore DIOR^{™}.

Trois échantillons ont été étudiés.
- le premier échantillon est la composition C2 préparée comme décrit à l'Exemple 3 à partir du support binaire L1 + L2, imprégné du parfum;
- le deuxième échantillon est le parfum déposé sur un support ne comprenant que le lot de particules poreuses L1;
- le troisième échantillon est le parfum déposé sur un support ne comprenant que le lot de particules pelliculaires L2.

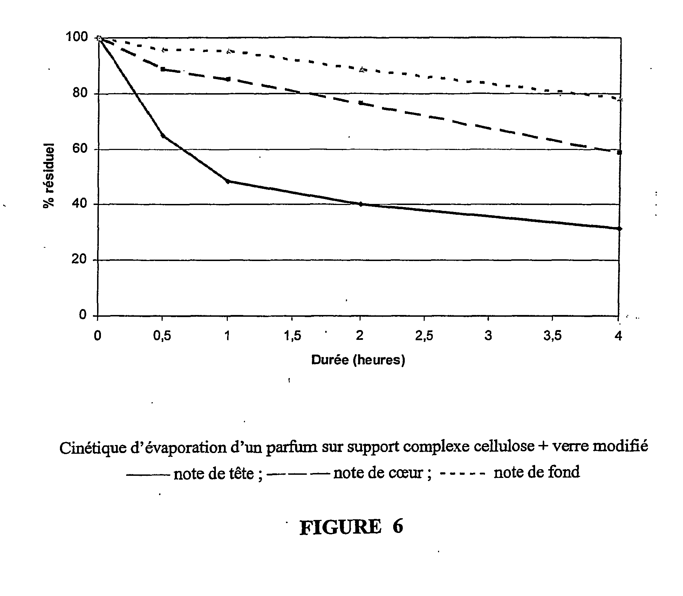

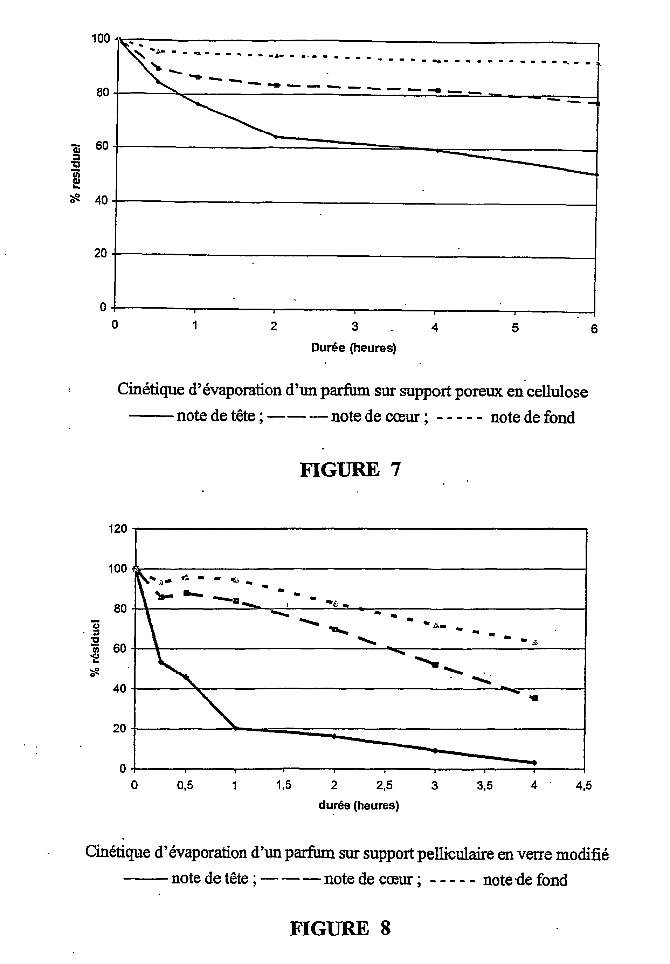

Les résultats obtenus sont présentés sur les figures 6, 7 et 8. Pour la composition binaire C2, les trois notes diffusent progressivement et sont encore présentes après huit heures (Figure 6). La contribution des supports poreux et pelliculaire à la diffusion contrôlée du parfum est illustrée par les Figures 7 et 8, respectivement.

## Revendications

1. Composition parfumée sèche à diffusion contrôlée ***caractérisée en ce qu'**elle* comprend:
a) un parfum, et
b) un support complexe susceptible de se charger en ledit parfum, comprenant en mélange au moins :
- i) un premier lot de particules, dites particules poreuses, ayant un diamètre moyen compris entre 10 µm et 500 µm, dont au moins 80% du volume est poreux, et
- ii) un deuxième lot de particules, dites particules pelliculaires, ayant un diamètre moyen compris entre 10 µm et 500 µm, dont seule une couche externe représentant au plus 20% du volume est poreuse.

2. Composition selon la revendication 1 ***caractérisée en ce que*** les particules poreuses dudit premier lot ont un diamètre compris entre 50 µm et 200 µm.

3. Composition selon la revendication 2 ***caractérisée en ce que*** les particules poreuses dudit premier lot ont une surface spécifique comprise entre 100 m²/g et 800 m²/g, de préférence entre 300 m²/g et 800 m²/g, et encore de préférence entre 500 m²/g et 800 m²/g.

4. Composition selon la revendication 1 ***caractérisée en ce que*** les particules pelliculaires dudit deuxième lot ont un diamètre compris entre 15 µm et 150 µm.

5. Composition selon la revendication 4 ***caractérisée en ce que*** les particules pelliculaires dudit deuxième lot ont une surface spécifique comprise entre 1 m²/g et 100 m²/g, de préférence entre 10 m²/g et 100 m²/g.

6. Composition selon l'une quelconque des revendications précédentes ***caractérisée en ce que*** les particules composant ledit support complexe sont hydrophobes.

7. Composition selon la revendication précédente ***caractérisé en ce que*** les particules composant ledit support sont choisies parmi les billes de silice poreuse, les billes de verre modifiées, les fibres de verre, les particules de cellulose ou les fibres de cellulose.

8. Composition selon l'une des revendications 1 à 7 ***caractérisée en ce que*** ledit support complexe comprend ledit premier lot de particules poreuses et ledit deuxième lot de particules pelliculaires dans un rapport en poids compris entre 1/1 et 1/50, de préférence entre 1/5 et 1/30, de manière encore préférée entre 1/10 et 1/20.

9. Composition selon l'une des revendications précédentes ***caractérisée en ce qu'***elle comprend de 10 à 90%, de préférence de 20 à 80%, de manière encore préférée de 40 à 75% de support, en poids rapporté au poids total de la composition.

10. Composition selon l'une des revendications précédentes ***caractérisée en ce qu'***elle comprend de 10 à 90%, de préférence de 20 à 80 %, de manière encore préférée de 25 à 60% de parfum, en poids rapporté au poids total de la composition.

11. Composition selon l'une des revendications 7 ou 8 ***caractérisée en ce que**,* dix minutes environ après la réalisation de la composition, le parfum comprend moins de 25%, de préférence moins de 15%, et encore de préférence moins de 5% d'un solvant volatile, en poids rapporté au poids initial de parfum.

12. Composition selon l'une quelconque des revendications précédentes ***caractérisés en ce que*** le parfum est choisi parmi un extrait naturel brut, une huile essentielle, une composition parfumée naturelle, une composition parfumée synthétique ou un mélange de ceux-ci.

13. Procédé de préparation d'une composition parfumée sèche, ***caractérisé en ce qu'***il comprend essentiellement les étapes consistant à:
- préparer un support complexe en mélangeant:
- i) un premier lot de particules, dites particules poreuses, ayant un diamètre moyen compris entre 10 µm et 500 µm, dont au moins 80% du volume est poreux, et
- ii) un deuxième lot de particules, dites particules pelliculaires, ayant un diamètre moyen compris entre 10 µm et 500 µm, dont seule une couche externe représentant au plus 20% du volume est poreuse,
- verser un parfum sur ledit support,
- laisser le parfum imprégner le support quelques minutes à température ambiante, et
- placer la composition parfumée sèche ainsi obtenue dans un réceptacle adapté au stockage ou à une utilisation immédiate.

14. Procédé selon la revendication 13 ***caractérisé en ce que*** la préparation dudit support complexe est réalisée à l'aide de deux lots de particules aptes à la préparation d'une composition selon l'une des revendications 2 à 7.

15. Procédé selon l'une des revendications 13 ou 14 ***caractérisé en ce que*** ledit support complexe est obtenu par mélange dudit premier lot de particules poreuses et dudit deuxième lot de particules pelliculaires dans un rapport en poids compris entre 1/1 et 1/50, de préférence entre 1/5 et 1/30, de manière encore préférée entre 1/10 et 1/20.

16. Procédé selon l'une des revendications 13 à 15 ***caractérisé en ce que*** ladite composition est obtenue par imprégnation dudit parfum sur ledit support poreux,
- ledit support constituant de 10 à 90%, de préférence de 20 à 80%, de manière encore préférée de 40 à 75%, en poids rapporté au poids total de la composition,
- ledit parfum constituant de 10 à 90%, de préférence de 20 à 80 %, de manière encore préférée de 25 à 60% de parfum, en poids rapporté au poids total de la composition, et le parfum contenant deux minutes après la préparation de la composition, moins de 25%, de préférence moins de 15%, et encore de préférence moins de 5% d'un solvant volatile, en poids de solvant rapporté au poids total de la composition.

17. Procédé selon la revendications 16 ***caractérisé en ce que*** la proportion de parfum et de support est telle que, après l'étape d'imprégnation, la composition se présente sous la forme d'une poudre sèche odorante.

18. Dispositif destiné à la préparation extemporanée d'une composition parfumée sèche à diffusion contrôlée, ***caractérisé en ce qu'***il comprend:
- une pluralité de premiers récipients contenant un parfum, et
- un pluralité de deuxièmes récipients contenant un support complexe susceptible de se charger en ledit parfum, comprenant en mélange au moins :
- i) un premier lot de particules, dites particules poreuses, ayant un diamètre moyen compris entre 10 µm et 500 µm, dont au moins 80% du volume est poreux, et
- ii) un deuxième lot de particules, dites particules pelliculaires, ayant un diamètre moyen compris entre 10 µm et 500 µm, dont seule une couche externe représentant au plus 20% du volume est poreuse.

19. Dispositif selon la revendication 18, ***caractérisé en ce que*** lesdits premiers et deuxièmes récipients contiennent respectivement les composants a) et b) d'une composition parfumée sèche selon l'une des revendications 1 à 12.

20. Dispositif selon la revendication 18, ***caractérisé en ce que*** lesdits premiers et deuxièmes récipients contiennent respectivement les quantités de parfum et de support nécessaires à la préparation extemporanée d'une dose de ladite composition sèche parfumée.

21. Application de la composition selon l'une des revendications 1 à 12 pour parfumer la peau, les cheveux, les vêtements, les accessoires vestimentaires, les bijoux, l'atmosphère d'un local.

## Claims

1. Dry scented composition with controlled dissemination ***characterised in that** it* includes:
a) a scent, and
b) a complex medium capable of being charged with the said scent, including a mix of at least:
-i) a first batch of particles, said to be porous, with a mean diameter of between 10 µm and 500 µm, including at least 80% of porous volume, and
-ii) a second batch of particles, said to be pellicular particles, with a mean diameter of between 10 µm and 500 µm, of which only a single external layer representing at most 20% of the volume is porous.

2. Composition according to claim 1 ***characterised in that*** the porous particles of the said first batch have a diameter of between 50 µm and 200 µm.

3. Composition according to claim 2 ***characterised in that*** the porous particles of the said first batch have a specific surface between 100 m²/g and 800 m²/g, preferably between 300 m²/g and 800 m²/g, and even more preferably between 500 m²/g and 800 m²/g.

4. Composition according to claim 1 ***characterised in that*** the pellicular particles of the said second batch have a diameter of between 15 µm and 150 µm.

5. Composition according to claim 4 ***characterised in that*** the pellicular particles of the said second batch have a specific surface of between 1 m²/g and 100 m²/g, and preferably between 10 m²/g and 100 m²/g.

6. Composition according to any of the previous claims ***characterised in that*** the particles making up the said complex medium are hydrophobic...

7. Composition according to the previous claim ***characterised in that*** the particles making up the said medium are chosen from among porous silica beads, modified glass beads, glass fibres, cellulose particles or cellulose fibres.

8. Composition according to one of claims 1 to 7 ***characterised in that*** the said complex medium includes the said first batch of porous particles and the said second batch of pellicular particles in a weight ratio of between 1/1 and 1/50, preferably between 1/5 and 1/30, or even more preferably between 1/10 and 1/20.

9. Composition according to one of the previous claims ***characterised in that*** it includes from 10 to 90%, preferably from 20 to 80%, and even more preferably from 40 to 75% of medium, in weight related to the overall weight of the composition.

10. Composition according to one of the previous claims ***characterised in that*** it includes from 10 to 90%, preferably from 20 to 80%, and even more preferably from 25 to 60% of scent, in weight related to the overall weight of the composition.

11. Composition according to one of claims 7 or 8 ***characterised in that*** about ten minutes after production of the composition, the scent includes less than 25%, preferably less than 15%, and even more preferably less than 5% of volatile solvent, in weight related to the initial weight of the scent.

12. Composition according to any of the previous claims ***characterised in that*** the scent is chosen from among a raw natural extract, an essential oil, a natural scented composition, a synthetic scented composition or a mixture of these.

13. Preparation process for a dry scented composition, ***characterised in that*** it mainly includes stages involving:
- preparing a complex medium combining:
-i) a first batch of particles, said to be porous, with a mean diameter of between 10 µm and 500 µm, including at least 80% of porous volume, and
-ii) a second batch of particles, said to be pellicular particles, with a mean diameter of between 10 µm and 500 µm, of which only a single external layer representing at most 20% of the volume is porous.
- pouring a scent onto the said medium,
- leaving the scent to impregnate the medium for a few minutes at ambient temperature, and
- placing the dry scented composition thus obtained in a receptacle suited to storage or to immediate use.

14. Process according to claim 13 ***characterised in that*** preparation of the said medium is achieved using two batches of particles suited for the preparation of a composition according to one of claims 2 to 7.

15. Process according to one of claims 13 or 14 ***characterised in that*** the said complex medium is obtained by a mixture of the said first batch of porous particles and of the said second batch of pellicular particles in a weight ratio between 1/1 and 1/50, preferably between 1/5 and 1/30, and even more preferably between 1/10 and 1/20.

16. Process according to one of claims 13 or 14 ***characterised in that*** the said composition is obtained by impregnation of the said scent on the said porous medium,
- the said medium constituting from 10 to 90%, preferably from 20 to 80%, and even more preferably from 40 to 75% in weight in relation to the overall weight of the composition,
- the said scent constituting from 10 to 90%, preferably from 20 to 80%, and even more preferably from 25 to 60% of scent in weight in relation to the overall weight of the composition, and the scent containing less than 25%, preferably less than 15% and even more preferably less than 5% of volatile solvent in weight of solvent in relation to the overall weight of the composition two minutes after preparation of the composition.

17. Process according to claim 16 ***characterised in that*** the proportion of scent and medium is such that, after the impregnation stage, the composition appears in the form of a scented dry powder.

18. Device intended for an extemporaneous preparation of a dry scented preparation with controlled dissemination, ***characterised in that*** it includes:
- a plurality of first recipients containing a scent, and
- a plurality of second recipients containing a medium capable of being charged with the said scent, including a mix of at least:
-i) a first batch of particles, said to be porous, with a mean diameter of between 10 µm and 500 µm, including at least 80% of porous volume, and
-ii) a second batch of particles, said to be pellicular particles, with a mean diameter of between 10 µm and 500 µm, of which only a single external layer representing at most 20% of the volume is porous.

19. Device according to claim 18, ***characterised in that*** the said first and second recipients contain respectively components a) and b) of a dry scented composition according to one of claims 1 to 12.

20. Device according to claim 18, ***characterised in that*** the said first and second recipients contain respectively quantities of scent and medium needed for the extemporaneous preparation of a dose of the said dry scented composition.

21. Application of the composition according to one of claims 1 to 12 to scent water, hair, clothes, clothing accessories, jewellery and the atmosphere in a room.

## Patentansprüche

1. Trockene duftstoffhaltige Zusammensetzung zur kontrollierten Abgabe, ***dadurch gekennzeichnet, dass*** sie Folgendes umfasst:
a) einen Duftstoff und
b) einen Trägerstoff komplexer Zusammensetzung, der den Duftstoff aufnehmen kann und mindestens Folgendes in Mischung umfasst:
- i) eine erste Charge von Partikeln, die als poröse Partikel bezeichnet werden, einen mittleren Durchmesser von 10 µm bis 500 µm haben und von deren Volumen mindestens 80 % porös ist, und
- ii) eine zweite Charge von Partikeln, die als Schalenpartikel bezeichnet werden und einen mittleren Durchmesser von 10 µm bis 500 µm haben, wobei nur deren äußere Schicht, welche höchstens 20 % des Volumens ausmacht, porös ist.

2. Zusammensetzung gemäß dem Anspruch 1, ***dadurch gekennzeichnet, dass*** die porösen Partikel der ersten Charge einen Durchmesser von 50 µm bis 200 µm aufweisen.

3. Zusammensetzung gemäß dem Anspruch 2, ***dadurch gekennzeichnet, dass*** die porösen Partikel der ersten Charge eine spezifische Oberfläche von 100 m²/g bis 800 m²/g, vorzugsweise von 300 m²/g bis 800 m²/g und insbesondere von 500 m²/g bis 800 m²/g aufweisen.

4. Zusammensetzung gemäß dem Anspruch 1, ***dadurch gekennzeichnet, dass*** die Schalenpartikel der zweiten Charge einen Durchmesser von 15 µm bis 150 µm aufweisen.

5. Zusammensetzung gemäß dem Anspruch 4, ***dadurch gekennzeichnet, dass*** die Schalenpartikel der zweiten Charge eine spezifische Oberfläche von 1 m²/g bis 100 m²/g, vorzugsweise von 10 m²/g bis 100 m²/g aufweisen.

6. Zusammensetzung gemäß einem beliebigen der vorhergehenden Ansprüche, ***dadurch gekennzeichnet, dass*** die Partikel, aus denen der Trägerstoff komplexer Zusammensetzung besteht, hydrophob sind.

7. Zusammensetzung gemäß dem vorhergehenden Anspruch, ***dadurch gekennzeichnet, dass*** die Partikel, aus denen sich der Trägerstoff zusammensetzt, aus porösen Siliziumdioxid-Kügelchen, modifizierten Glaskügelchen, Glasfasern, Cellulosepartikeln oder Cellulosefasern ausgewählt sind.

8. Zusammensetzung gemäß einem der Ansprüche 1 bis 7, ***dadurch gekennzeichnet, dass*** der Trägerstoff komplexer Zusammensetzung die erste Charge aus porösen Partikeln und die zweite Charge aus Schalenpartikeln in einem Gewichtsverhältnis umfasst, das zwischen 1/1 und 1/50, vorzugsweise zwischen 1/5 und 1/30 und insbesondere zwischen 1/10 und 1/20 liegt.

9. Zusammensetzung gemäß einem der vorhergehenden Ansprüche, ***dadurch gekennzeichnet, dass*** sie 10 bis 90 Gewichts%, vorzugsweise 20 bis 80 Gewichts% und insbesondere 40 bis 75 Gewichts% an Trägerstoff umfasst, bezogen auf das Gesamtgewicht der Zusammensetzung.

10. Zusammensetzung gemäß einem der vorhergehenden Ansprüche, ***dadurch gekennzeichnet, dass*** sie 10 bis 90 Gewichts%, vorzugsweise 20 bis 80 Gewichts% und insbesondere 25 bis 60 Gewichts% an Duftstoff umfasst, bezogen auf das Gesamtgewicht der Zusammensetzung.

11. Zusammensetzung gemäß einem der Ansprüche 7 oder 8, ***dadurch gekennzeichnet, dass*** der Duftstoff ungefähr zehn Minuten nach der Herstellung der Zusammensetzung weniger als 25 Gewichts%, vorzugsweise weniger als 15 Gewichts% und insbesondere weniger als 5 Gewichts% eines flüchtigen Lösemittels umfasst, bezogen auf das Ursprungsgewicht des Duftstoffs.

12. Zusammensetzung gemäß einem beliebigen der vorhergehenden Ansprüche, ***dadurch gekennzeichnet, dass*** der Duftstoff aus einem rohen natürlichen Extrakt, einem ätherischen Öl, einer natürlichen duftstoffhaltigen Zusammensetzung, einer synthetischen duftstoffhaltigen Zusammensetzung oder einer Mischung daraus gewählt ist.

13. Verfahren zur Herstellung einer trockenen duftstoffhaltigen Zusammensetzung, ***dadurch gekennzeichnet, dass*** es im Wesentlichen die folgenden Schritte umfasst:
- einen Trägerstoffs komplexer Zusammensetzung herstellen, indem Folgendes vermischt wird:
- i) eine erste Charge von Partikeln, die als poröse Partikel bezeichnet werden, einen mittleren Durchmesser von 10 µm bis 500 µm haben und von deren Volumen mindestens 80 % porös ist, und
- ii)eine zweite Charge von Partikeln, die als Schalenpartikel bezeichnet werden und einen mittleren Durchmesser von 10 µm bis 500 µm haben, wobei nur deren äußere Schicht, welche höchstens 20 % des Volumens ausmacht, porös ist,
- einen Duftstoff auf den Trägerstoff gießen,
- den Duftstoff bei Raumtemperatur einige Minuten lang den Trägerstoff durchtränken lassen, und
- die auf diese Weise erhaltene trockene duftstoffhaltige Zusammensetzung in ein Behältnis geben, welches sich zur Lagerung oder zur sofortigen Verwendung eignet.

14. Verfahren gemäß dem Anspruch 13, ***dadurch gekennzeichnet, dass*** die Herstellung des Trägerstoffs komplexer Zusammensetzung mit Hilfe zweier Chargen von Partikeln erfolgt, welche sich für die Herstellung einer Zusammensetzung gemäß einem der Ansprüche 2 bis 7 eignen.

15. Verfahren gemäß einem der Ansprüche 13 oder 14, ***dadurch gekennzeichnet, dass*** der Trägerstoff komplexer Zusammensetzung durch Vermischen der ersten Charge aus porösen Partikeln und der zweiten Charge aus Schalenpartikeln erhalten wird, wobei deren Gewichtsverhältnis zwischen 1/1 und 1/50, vorzugsweise zwischen 1/5 und 1/30 und insbesondere zwischen 1/10 und 1/20 liegt.

16. Verfahren gemäß einem der Ansprüche 13 bis 15, ***dadurch gekennzeichnet, dass*** die Zusammensetzung erhalten wird, indem der poröse Trägerstoff mit dem Duftstoff durchtränkt wird,
- wobei der Trägerstoff 10 bis 90 Gewichts%, vorzugsweise 20 bis 80 Gewichts% und insbesondere 40 bis 75 Gewichts% ausmacht, bezogen auf das Gesamtgewicht der Zusammensetzung,
- wobei der Duftstoff 10 bis 90 Gewichts%, vorzugsweise 20 bis 80 Gewichts% und insbesondere 25 bis 60 Gewichts% ausmacht, bezogen auf das Gesamtgewicht der Zusammensetzung, und wobei der Duftstoff zwei Minuten nach der Herstellung der Zusammensetzung weniger als 25 Gewichts%, vorzugsweise weniger als 15 Gewichts% und insbesondere weniger als 5 Gewichts% eines flüchtigen Lösemittel enthält, bezogen auf das Gesamtgewicht der Zusammensetzung.

17. Verfahren gemäß dem Anspruch 16, ***dadurch gekennzeichnet, dass*** die Mengenanteile an Duftstoff und an Trägerstoff derart sind, dass die Zusammensetzung nach dem Durchtränkungsschritt in Form eines duftenden, trockenen Pulvers vorliegt.

18. Vorrichtung, die dazu bestimmt ist, eine trockene duftstoffhaltige Zusammensetzung zur kontrollierten Abgabe unmittelbar vor deren Einsatz herzustellen, ***dadurch gekennzeichnet, dass*** sie Folgendes umfasst:
- mehrere erste Behältnisse, die einen Duftstoff enthalten, und
- Mehrere zweite Behältnisse, die einen Trägerstoff komplexer Zusammensetzung enthalten, welcher den Duftstoff aufnehmen kann und mindestens Folgendes in Mischung umfasst:
- i) eine erste Charge von Partikeln, die als poröse Partikel bezeichnet werden, einen mittleren Durchmesser von 10 µm bis 500 µm haben und von deren Volumen mindestens 80 % porös ist, und
- ii) eine zweite Charge von Partikeln, die als Schalenpartikel bezeichnet werden und einen mittleren Durchmesser von 10 µm bis 500 µm haben, wobei nur deren äußere Schicht, welche höchstens 20 % des Volumens ausmacht, porös ist.

19. Vorrichtung gemäß dem Anspruch 18, ***dadurch gekennzeichnet, dass*** die ersten und zweiten Behältnisse die Bestandteile a) beziehungsweise b) einer trockenen, duftstoffhaltigen Zusammensetzung gemäß einem der Ansprüche 1 bis 12 enthalten.

20. Vorrichtung gemäß dem Anspruch 18, ***dadurch gekennzeichnet, dass*** die ersten und zweiten Behältnisse diejenigen Mengen an Duftstoff beziehungsweise an Trägerstoff enthalten, die dafür erforderlich sind, eine Dosis der trockenen duftstoffhaltigen Zusammensetzung unmittelbar vor deren Einsatz herzustellen..

21. Anwendung der Zusammensetzung gemäß einem der Ansprüche 1 bis 12, um der Haut, dem Haar, der Kleidung, Kleidungsaccessoires, Schmuck oder der Raumluft einen Duft zu verleihen.
